# EUROPEAN PATENT SPECIFICATION

(11) **EP 2 938 253 B1**
(45) Date of publication and mention of the grant of the patent: **24.03.2021**
(21) Application number: 13868631.6
(22) Date of filing: 23.12.2013
(51) Int. Cl.: A61B 5/0215, A61B 5/00

(54) **GUIDEWIRE WITH AN IN-WALL HYPOTUBE SENSOR MOUNT AND THE ASSOCIATED MANUFACTURING METHOD**
FÜHRUNGSDRAHT MIT WANDINTERNE HYPOROHRSENSORHALTERUNG UND DAS ZUGEHÖRIGE HERSTELLUNGSVERFAHREN
FIL-GUIDE AVEC CAPTEUR MONTÉ DANS UN HYPOTUBE ET LA MÉTHODE DE FABRICATION ASSOCIÉE

(30) Priority: 31.12.2012 US 201261747573 P
(43) Date of publication of application: 04.11.2015
(73) Proprietor: Philips Image Guided Therapy Corporation, San Diego CA 92130 (US)
(72) Inventor: BURKETT, David H., Temecula, California 92591 (US)
(74) Representative: de Haan, Poul Erik
(86) International application number: PCT/US2013/077406
(87) International publication number: WO 2014/105785

(56) References cited:
- WO-A1-96/07351
- WO-A1-2012/109039
- JP-A- 2001 170 013
- US-A- 5 873 835
- US-A1- 2003 220 588
- US-A1- 2004 242 990
- US-A1- 2010 210 923
- US-A1- 2010 241 008
- US-A1- 2012 220 883
- US-B2- 8 317 715

## Description

### TECHNICAL FIELD

The present disclosure relates to intravascular systems and methods that include a hypotube having an integrated sensor mount.

### BACKGROUND

With the advent of angioplasty, pressure measurements have been taken in vessels and particularly in coronary arteries for the treatment of certain ailments or conditions. Typically in the past, such pressure measurements have been made by measuring the pressure at a proximal extremity of a lumen provided in a catheter advanced into the coronary artery of interest. Such an approach has, however, been less efficacious as the diameters of the catheters became smaller with the need to advance the catheter into smaller vessels and to the distal side of atherosclerotic lesions. This made necessary the use of smaller lumens that gave less accurate pressure measurements and in the smallest catheters necessitated the elimination of such a pressure lumen entirely. Furthermore, catheters are often large enough to significantly interfere with the blood flow and damp the pressure resulting in an inaccurate pressure measurement. In an attempt to overcome these difficulties, ultra miniature pressure sensors have been proposed for use on the distal extremities of a guidewire. Using a guidewire with a smaller diameter is less disruptive to the blood flow and thus provides a more accurate pressure reading.

However the manufacturing process to consistently locate miniature sensors in guidewires can be challenging. For example, because of their size, current sensors on guide wires are mounted by hand in a cutout or mounted along a core wire. However, the optimal alignment of the sensor is dependent upon an assembler's ability to align the sensor within a given design. Because the sensors are placed by hand, there is frequently some variability in sensor location from guidewire to guidewire. This variability may be compounded when sensors are located or placed by different workers.

US 2010/0241008 A1 describes a catheter with a guidewire. The catheter comprising a guiding tube for sliding the guidewire therethrough and a tip pressure sensor eccentrically mounted relative to the guiding tube.

WO 96/07351 A1 describes a guidewire having pressure sensing capabilities. A flexible elongate member of the guidewire carries a housing and a pressure sensor is mouted in the housing space.

JP 2001/170013 A describes a catheter with pressure detecting function, the catheter including a sensor support block with a sensor chip housing recess having a substantially rectangular shape. The pressure sensor chip is fixed to a mounting surface in the sensor chip housing recess using an epoxy or silicone resin adhesive.

Accordingly, there remains a need for improved devices, systems, and methods that have a capacity for increased consistency among workers even when the systems, devices, and methods are performed by hand. The present disclosure addresses one or more of the problems in the prior art.

### SUMMARY

The invention is defined in the independent claims 1 and 8. Preferred embodiments are set forth in the dependent claims.

The present disclosure is directed to a guidewire system for treating a patient. The guidewire system includes a sensor assembly for detecting a physiological characteristic of a patient and a hypotube sized for insertion into vasculature of the patient. The hypotube has an integrated sensor mount formed therein for predictably locating the sensor during assembly. The hypotube has a wall structure and a lumen, and the sensor mount is formed within the wall structure of the hypotube and includes a first mechanical stop configured to limit movement of the sensor assembly in at least a first dimension and a second mechanical stop configured to limit movement of the sensor assembly in at least a second dimension. A sensor housing is disposed about the sensor mount and has a window formed therein to provide fluid communication between the sensor assembly and an environment outside the hypotube.

In an aspect, the sensor mount comprises a shelf formed by the wall structure of the hypotube, the shelf being radially displaced from the outer surface of the hypotube. In an aspect, the sensor mount comprises a through passage adjacent the shelf, the through passage being sized and configured to accommodate a portion of the sensor assembly extending into a lumen of the hypotube when the sensor assembly is on the sensor mount. In an aspect, the second mechanical stop comprises lateral wall structure of the hypotube adjacent the sensor assembly. According to the invention, the integrated sensor mount has a width sized to receive the sensor assembly and limit the lateral movement of the sensor assembly. The integrated sensor mount has a first region having a first width and a second region having a greater second width, the sensor assembly having a width corresponding to the first width of the first region in a manner that substantially limits movement in a first direction, the sensor assembly extending into the second region, the second width providing additional clearance for the sensor assembly in a manner that prevents distortion of sensor readings when the hypotube flexes as it traverses tortious vessels in the patient. According to the invention, the first mechanical stop is configured to maintain the sensor at a desired height and the second mechanical stop is configured to maintain the sensor at a desired lateral location. In an aspect, the sensor housing increases rigidity of the hypotube at the sensor mount. In an aspect, the integrated sensor mount comprises a cutout having a first level and a second level, the sensor assembly being disposed on the first level, the second level being lower than the first level. In an aspect, a portion of the sensor assembly extends off the shelf to a cantilevered position within the sensor mount. In an aspect, the hypotube is formed of Nitinol. In an aspect, the sensor housing is formed of stainless steel.

In an aspect, the sensor mount is a first sensor mount, the guidewire system comprising a second sensor mount and a second sensor assembly.

Furthermore, the present disclosure is directed to a method of building a guidewire including providing a hypotube sized for introduction to a patient's vasculature when treating a medical condition, the hypotube having a sensor mount therein, the sensor mount formed within the wall structure of the hypotube and having a first mechanical stop configured to limit movement of the sensor assembly in at least a first dimension and a second mechanical stop configured to limit movement of the sensor assembly in at least a second dimension, wherein the sensor mount has a first region having a first width forming the second mechanical stop and a second region having a greater second width; radially introducing a sensor assembly into the sensor mount in the hypotube, the sensor assembly having a width corresponding to the first width of the first region of the sensor mount, until the sensor assembly abuts a shelf recessed in a wall structure of the hypotube to locate the sensor assembly at a pre-established height by limiting movement of the sensor assembly beyond the shelf in the radial direction, wherein radially introducing the sensor assembly also includes aligning the sensor assembly with the sensor mount so that lateral movement is substantially prevented in the first region of the sensor mount and so that the sensor assembly extends into the second region, the second width providing additional clearance for the sensor assembly, in order to obtain consistency in sensor assembly placement from one guidewire to another.

In an aspect, the method includes extending a portion of the sensor assembly through a passage of the hypotube and into a lumen of the hypotube. In an aspect, the portion of the sensor assembly is a plurality of conductors configured to communicate signals from a sensor to a proximal end of the guidewire. In an aspect, the method includes introducing the sensor mount into a sensor housing having a window formed therein to provide fluid communication between the sensor and an environment outside the sensor housing.

### BRIEF DESCRIPTION OF THE DRAWINGS

Illustrative embodiments of the present disclosure will be described with reference to the accompanying drawings, of which:
Fig. 1 illustrates a diagrammatic side view of a guidewire system according to an exemplary embodiment of the present disclosure.
Fig. 2 illustrates a diagrammatic side view of a guidewire according to an exemplary embodiment of the present disclosure.
Fig. 3 illustrates a side view of a distal region of the guidewire of Fig. 2 according to an exemplary aspect of the present disclosure.
Fig. 4 illustrates a cross-sectional view of a portion of the distal region of the guidewire of Fig. 2 according to an exemplary aspect of the present disclosure.
Fig. 5 illustrates an isometric view of a sensor assembly according to an exemplary aspect of the present disclosure.
Fig. 6 illustrates a portion of a hypotube with an integrated sensor mount according to an exemplary aspect of the present disclosure.
Fig. 7 illustrates a cross-sectional side view of the hypotube of Fig. 6 according to an exemplary aspect of the present disclosure.
Fig. 8 illustrates a top view of the hypotube of Fig. 6 according to an exemplary aspect of the present disclosure.
Fig. 9 illustrates a cross-sectional view taken along lines 9-9 of the hypotube of Fig. 8 according to an exemplary aspect of the present disclosure.
Fig. 10 illustrates a cross-sectional view taken along lines 10-10 of the hypotube of Fig. 8 according to an exemplary aspect of the present disclosure.
Fig. 11 illustrates a cross-sectional view taken along lines 11-11 of the hypotube of Fig. 8 according to an exemplary aspect of the present disclosure.
Fig. 12 illustrates a portion of a hypotube with a sensor assembly in an integrated sensor mount according to an exemplary aspect of the present disclosure.
Fig. 13 illustrates a cross-sectional side view of the hypotube and sensor assembly of Fig. 12 according to an exemplary aspect of the present disclosure.
Fig. 14 illustrates a cross-sectional side view of a hypotube and an alternative sensor assembly according to an exemplary aspect of the present disclosure.
Fig. 15 illustrates a cross-sectional side view of a hypotube having a plurality of sensor mounts and a plurality of sensor assemblies according to an exemplary aspect of the present disclosure.

### DETAILED DESCRIPTION

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any connections and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

The devices, systems, and methods disclosed herein include a guidewire with an integrated sensor mount that is configured to increase the repeatability and consistency of sensor placement during the manufacturing process. The sensor mount is arranged to enable a worker to locate the sensor at a precise height relative to the outer surfaces of the guidewire. The sensor mount is arranged to enable a worker to locate the sensor at a precise location in the lateral direction from the distal end of the guidewire. In some embodiments, the sensor mount is arranged to enable a worker to reference the sensor mount when placing the sensor to identify the axial position to increase consistency of assembly from guidewire to guidewire even among different workers. Some sensor mount embodiments allow a worker to locate the sensor in height, axial position, and lateral position. Accordingly, guidewires may be assembled with increased reliability and consistency. The guidewire having sensing capabilities may be adapted to be used in connection with a patient lying on a table or a bed in a cath lab of a typical hospital in which a catheterization procedure such as for diagnosis or treatment is being performed on the patient.

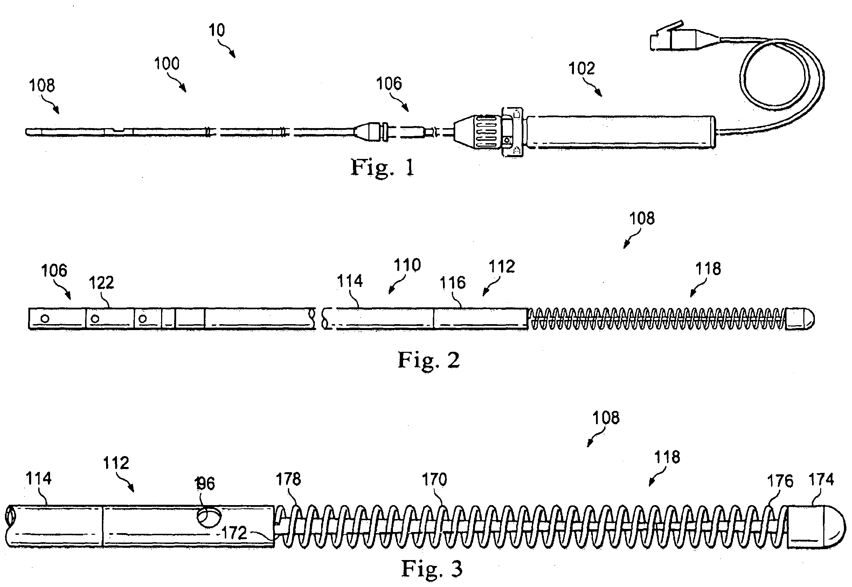

Fig. 1 shows an exemplary guidewire system 10 consistent with the principles disclosed herein. The guidewire system 10 in this embodiment is configured to sense or detect a physiological characteristic of a condition of the patent. For example, it may detect or sense a characteristic of the vasculature through which it has been introduced. In one embodiment, the guidewire system 10 has pressure sensing capabilities. The guidewire system 10 includes a guidewire 100 and a connector 102 disposed at the end of the guidewire 100. The connector 102 in this example in Fig. 1 is configured to communicate with the guidewire 100, serve as a grippable handle to enable the surgeon to easily manipulate the proximal end of the guidewire 100, and connect to a console or further system (not shown) with a modular plug. Accordingly, since the guidewire 100 is configured to detect physiological environmental characteristics, such as pressure in an artery for example, data or signals representing the detected characteristics may be communicated from the guidewire 100, through the connector 102, to a console or other system for processing. In this embodiment, the connector 102 is configured to selectively connect to and disconnect from the guidewire 100. In some embodiments, the guidewire system 10 is a single-use device. The guidewire 100, in the embodiment shown, is selectively attachable to the connector 102 and includes a proximal portion 106 connectable to the connector 102 and a distal portion 108 configured to be introduced to a patient during a surgical procedure.

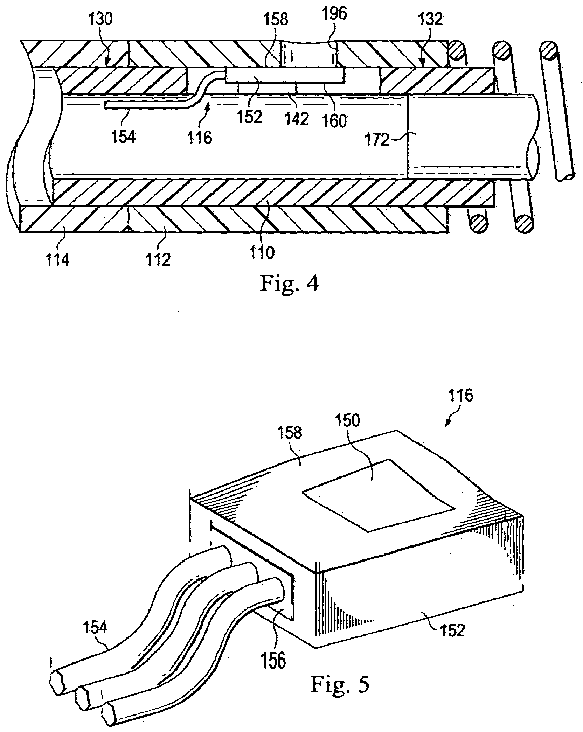

The guidewire 100 is shown in greater detail in Figs. 2-4. Fig. 2 shows the entire guidewire 100, Fig. 3 shows the distal portion 108 of the guidewire 100, and Fig. 4 shows a cross-section of a portion of the distal portion 108 of the guidewire 100. Referring to these Figures, the guidewire 100 includes a hypotube 110, a sensor housing 112, a proximal polymer sleeve 114, a sensor assembly 116, a distal tip 118 (Fig. 3), and a proximal electrical interface 122.

The proximal electrical interface 122 in Fig. 2 is configured to electrically connect the sensor assembly 116 and the connector 102 to order to ultimately communicate signals to the processing system. In accordance with this, the electrical interface 122 is in electrical communication with the sensor assembly 116 and in this embodiment is configured to be received within the connector 102. The electrical interface 122 may include a series of conductive contacts on its outer surface that engage and communicate with corresponding contacts on the connector 102.

The sensor assembly 116 is shown in Fig. 5 and includes a sensor 150 which in this embodiment includes a sensor diaphragm, a sensor block 152, and conductors 154 that extend from the sensor block 152 to the proximal electrical interface 122. The sensor 150 is arranged and configured to measure a physiological characteristic of a patient. When used on the guidewire 100, the sensor 150 is arranged and configured to measure a physiological characteristic of vasculature of the patient. In one embodiment, the sensor 150 is a pressure transducer configured to detect a pressure within a portion of a patient, such as the pressure within a blood vessel. In another embodiment, the sensor 150 is a flow control sensor that may be used to measure flow through the vessel. In yet other embodiments, the sensor 150 is a plurality of sensors arranged to detect one or more physiological characteristics of the patient and provide feedback or information relating to the detected physiological characteristic. The sensor 150 may be disposed, for example, less than about 5cm from the distal-most end of the guidewire 100. In one embodiment, the sensor is disposed about 3cm from the distal-most end of the guidewire 100.

The sensor block 152 carries the sensor 150 and may be, for example, a wafer, a chip, or other transducer carrying substrate. Depending on the sensor type, the sensor block 152 may include a cavity covered by a diaphragm of the pressure sensor to create the sensing portion of the sensor assembly 116. The sensor block 152 in this embodiment is configured to carry the sensor 150 and configured to have contacts 156 or conductive connectors for communication with the conductors 154. The sensor block 152 in this embodiment is sized to fit within the diametric profile of the guidewire 100. In the embodiment shown, the sensor block 152 is relatively rectangular shaped and includes an outwardly facing sensor side 158 and an inner facing side 160 (Fig. 4) that is configured to engage and directly lie against a portion of the sensor mount 134, discussed below. In this condition, the sensor block 152 may be particularly positioned in order to provide a consistent and predictable structure, reducing the chance of variation that may otherwise occur during manufacturing from employee to employee as the sensor block 152 is applied to the hypotube 110. The sensor block 152 may be sized to have an axial length in the range of about 0.508 to 1.397 mm ( 0.020 to 0.055 inch). In one embodiment, the axial length is about 0.889 mm (0.035 inch). The width may be in the range of about 0.1016 to 0.381 mm (0.004 to 0.015 inch). In one embodiment, the width is about 0.2286 mm (0.009 inch). The height may be in the range of about 0.0254 to 0.2032 mm (0.001 to 0.008 inch). In one embodiment, the height is about 0.0762 mm (0.003 inch). Other sizes of sensor blocks are contemplated. The contacts 156 on the sensor block 152 may be formed at the proximal end and may be shaped to communicate electrically with the conductors 154. In some embodiments, the contacts 156 are disposed along the inner facing side 160 of the sensor block 152 or on the outer facing side 158.

The conductors 154 extend from the contacts on the sensor block 152 to the proximal electrical interface 122 (Fig. 2). The conductors 154 are, in this embodiment, electrical cables or wires extending from the sensor block 152. In Fig. 4, the contacts 156 are disposed on the proximal end of the sensor block 152, and the conductors 154 bend to enter the inner lumen of the hypotube 110. However, other embodiments have the contacts 156 on the surfaces 158 or 160 with the conductors 154 appropriately connected. Since the guidewire 100 disclosed herein uses a hypotube 110, the system lacks a core and the conductors 154 can extend through the hypotube lumen. The example shown employs three conductors 154, however the number of conductors in any particular embodiment may depend in part on the type or number of sensors disposed within the guidewire 100. In some embodiments, the conductors 154 are soldered to the contacts 156 on the sensor block 152 during the manufacturing process. Accordingly, the conductors 154 may carry signals to and from the sensor 150.

The hypotube 110 is a flexible elongate element having a proximal end region 130 and a distal end region 132 which are formed of a suitable biocompatible material. The proximal end region 130 extends to the proximal electrical interface 122. In some embodiments, the hypotube 110 is formed of a Nitinol alloy, while in other embodiments, the hypotube is formed of stainless steel. Other materials would be apparent to one of ordinary skill in the art.

Some hypotube embodiments are large-diameter hypotubes having an outer diameter in the range of about, for example, 0.635 mm to 1.016 mm (0.025 inch to 0.040 inch). These may be used for peripheral vascular interventions for treating particular body regions of a patient. In some embodiments, the hypotube 110 has a diameter in the range of about 1.016 mm ( .040 inch) or less. Some embodiments have a 0.889 mm (0.035 inch) outer diameter. Some large-diameter hypotubes have an inner diameter sized to be about half of the outer diameter. For example, a 0.889 mm ( .035 inch) outer diameter may have an inner diameter of about 0.4064 mm (.016 inch) and a wall thickness of about. 0.2286 to 0.2540 mm (009 to .010 inch), for example. Hypotubes with other diameters and wall thicknesses are contemplated. In some examples, the hypotube 110 has an outside diameter for example of 0.4572 mm (0.018 inch) or less and has a suitable wall thickness of, for example, 0.1016 to 0.1270 mm ( 0.004 to 0.005 inch). Yet other sizes are also contemplated. In some embodiments, the hypotube has a length of about 150-200 centimeters, although other lengths are contemplated.

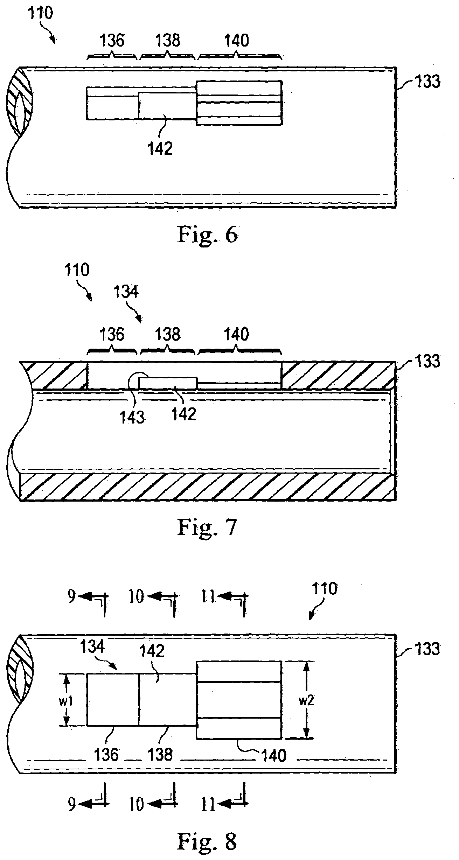

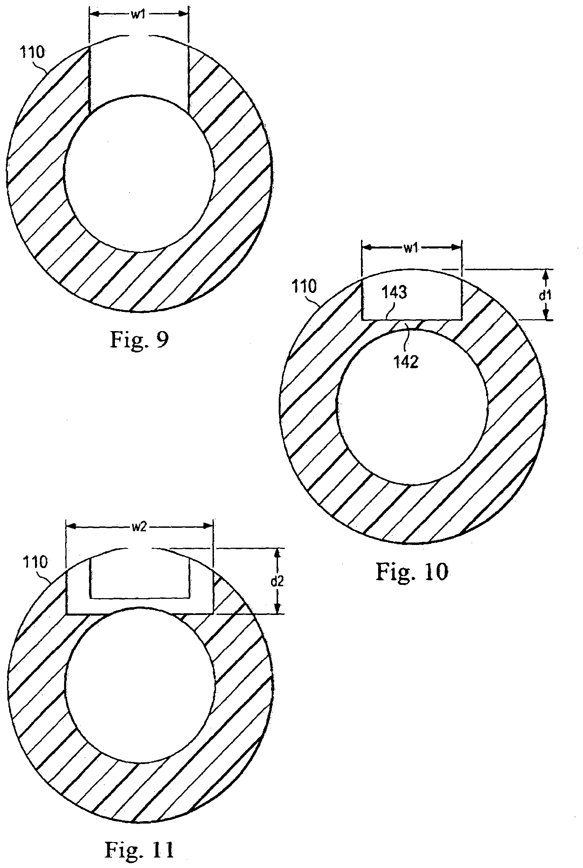

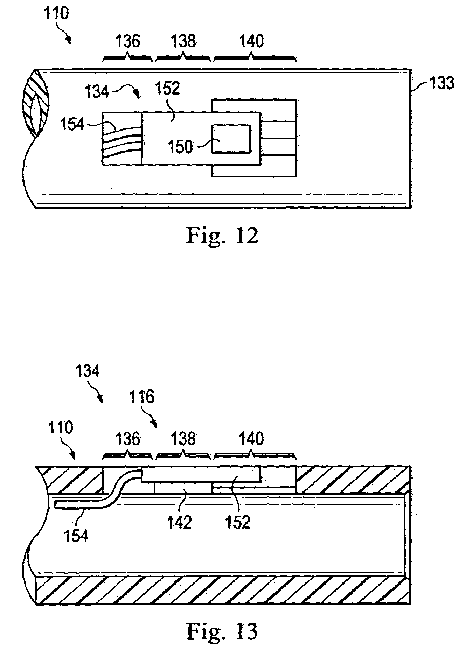

Fig. 6 shows the distal portion of the hypotube 110 including the integrated sensor mount 134. Fig. 7 shows the distal portion of the hypotube 110 taken in cross-section along the axis of the hypotube 110. Fig. 8 shows a top view of the hypotube 110 including the integrated sensor mount 134. Figs. 9, 10, and 11 are cross-sectional views taken through the hypotube 110 along the lines 9-9, 10-10, and 11-11, respectively. Figs. 12 and 13 show the distal portion of the hypotube 110 with the sensor assembly 116.

In this embodiment, as shown in Figs. 6 and 7, the hypotube 110 includes a distal end 133 and includes an integrated sensor mount 134 formed therein. Here the sensor mount 134 is a cut-out formed within a side of the hypotube 110 to receive at least a part of the sensor assembly 116. The sensor mount 134 is particularly sized and configured to help accurately align the sensor block 152 of the assembly 116 in the cutout. As discussed below, the geometry and size of the cutout as the sensor mount 134 can be used to precisely locate the sensor block 152 vertically (or in a first dimension) and laterally (or in a second dimension), while the ends of the sensor mount 134 provide a visual reference for aligning the sensor block 152 axially (or in a third dimension). Accordingly, the hypotube has an integral, built-in mounting feature. In addition, the hypotube diameter is designed to allow for a simpler external housing.

The sensor mount 134 may be disposed about; 2.54 cm (an inch) or less from the distal end 133 of the cylindrical portion of the hypotube 133. In one embodiment, the sensor mount is disposed about 3cm from a distal-most tip of the guidewire 100. In the embodiment shown in Figs. 6-11, the sensor mount 134 comprises a first region 136, a second region 138, and a third region 140.

The first region 136 in this case forms the proximal end of the sensor mount 134 and is disposed adjacent a completely enclosed or a completely cylindrical portion of the hypotube 110. The first region 136 is a through passage entirely through the wall of the hypotube from the outer surface of the hypotube to the inner surface forming the lumen of the hypotube 110. The first region is formed to accommodate the transmission carriers or conductors 154 that extend from the sensor block 152 to the proximal electrical interface 122. This may be seen in Figs. 12 and 13, where the conductors 154 extend from the distal end of the sensor block 152 and bend to enter the lumen of the hypotube 110. In some embodiments, the first region 136 starts at about 0.16 cm (0.0630 inch) from the distal end 133 and ends about 0.18 cm (0.0710 inch) from the distal end 133. However, other sizes and locations are contemplated. Fig. 9 shows a cross-sectional view of the hypotube 110 taken through the first region 136 of the sensor mount 134. As can be seen in Fig. 9, the first region 136 has a width w1, which is discussed further below.

The second region 138 is arranged to simplify the assembly of the guidewire 100 by guiding the placement of the sensor block 152 onto the hypotube 110. The second region 138 is disposed distal of the first region 136 and proximal of the third region 140. The second region 138 is formed to carry the sensor block 152 and acts as a mechanical stop that dictates the location or height of the sensor relative to the hypotube when the guidewire is assembled. To do this, the second region 138 includes a shelf 142 having an upper facing surface 143 that is radially displaced from or that has an elevation lower than that of the outer surface of the hypotube. In this example, the shelf 142 is formed by removing material from the wall of the hypotube 110 without breaking entirely through the wall. This is shown in cross-section in Fig. 10. The depth d1 of the second region 138 from the outer surface of the hypotube 110 to the shelf 142 may be selected to precisely orient the sensor block 152 at a pre-established height that may be an optimum height. For example, the depth d1 of the second region 138 may be within the range of about 0.0762 to 0.1524 mm (.0030" to .0060") and may be selected based on the height of the sensor block 152. Other sizes are contemplated.

In this embodiment, the width w1 of the sensor mount 134 in the second region 138 in Fig. 10 is selected to correspond roughly with the width of the sensor block 152 so that the sensor block 152 can be inserted into the second region 138 to lie directly on the shelf 142 with the sensor positioned at a desired height relative to the hypotube. Because the width w1 is selected to receive the sensor block 152 with little play in the lateral direction, the sensor assembly 152 may be placed within the sensor mount 134 so that the sensor is located with little variation in the height and with little variation in lateral position from hypotube to hypotube. Figs. 12 and 13 show the sensor block 152 disposed on the shelf 142 and disposed so that the lateral walls in the second region prevent lateral displacement when the sensor assembly is placed within the sensor mount 134. The shelf 142 and the lateral sides of the sensor mount 134 in the second region 138 enable workers to more easily place the sensor assembly in a consistent location when assembling the guidewires. As such, manufacturing efficiencies are achieved because workers may place the sensor assemblies directly against the shelf 142 in the second region 138 of the sensor mount 134 so that the height of the sensor assembly 116 is consistent across guidewires and so that the lateral position of the sensor assembly 116 is consistent across guidewires, increasing reliability, predictability of operation, and reproducibility. With the arrangement shown, the shelf 142 acts as a mechanical stop that maintains the sensor assembly at a proper height and the lateral walls act as mechanical steps that maintain the sensor assembly at a proper lateral direction.

In one embodiment, the second region 138 starts at about 1.1684 mm ( 0.0460 inch) from the distal end 133 and ends at about 1.60 mm (0.0630 inch) from the distal end 133 of the hypotube 110.

The third region 140 forms the distal end of the sensor mount 134 and is disposed adjacent a completely enclosed or a completely cylindrical portion 146 of the hypotube 110. In the exemplary embodiment shown, the third region 140 has a depth d2 greater than the depth d1 of the second region 138, forming steps with different levels. The depth d2 is greater than that of the depth d1 of the second region 138 to provide clearance for the sensitive end of the sensor assembly 116 so that the sensor block 152 is cantilevered within the sensor mount 134. This may be seen in Fig. 13, where the distal portion of the sensor block is cantilevered from the shelf 142. A cantilevered sensor block 152 may better isolate the sensor 150 from interference that may occur as a result of flexing of the hypotube 110 that may occur as the guidewire 100 is fed through a patient's vasculature. That is, while the hypotube 110 may flex, even along the sensor mount 134, the sensor readings may remain virtually unaffected because the sensor is cantilevered and therefore not subject to loading that may otherwise occur as a result of flexing of the hypotube 110.

In addition, as can be seen in Figs. 8, 10, and 11, the third region has a width w2 greater than the width w1. As such, the sensor block 152 does not contact or abut against the lateral walls of the third region 140 as shown in Fig. 12. Accordingly, when the hypotube 110 flexes in the lateral direction, the sensor readings are not affected by the displacement of the walls of the third region 140 adjacent the sensor because the sensor is spaced from the lateral walls. In addition, the sensor block 152 does not abut the distal end of the sensor mount 134 in order to isolate the sensor from flexing that may occur. However, some applications do not require such strict isolation and the spacing that isolates the sensor block may not be present.

In one embodiment, the third region starts about 0.381 mm (0.0150 inch ) from the distal end 133 of the hypotube 110 and ends about 1.1684 mm (0.0460 inch) from the distal end 133 of the hypotube 110. The distal cylindrical portion of the hypotube extends from the distal end 133 to about 0.381 mm (.0150") from the distal end 133. Other dimensions are contemplated.

In the embodiment shown, the third region 140 breaks through the wall structure to the lumen due to the thickness of the tubing. In some embodiments, the third region does not break through, while in other embodiments, the third region is formed as an entire passage, as is the first region 136.

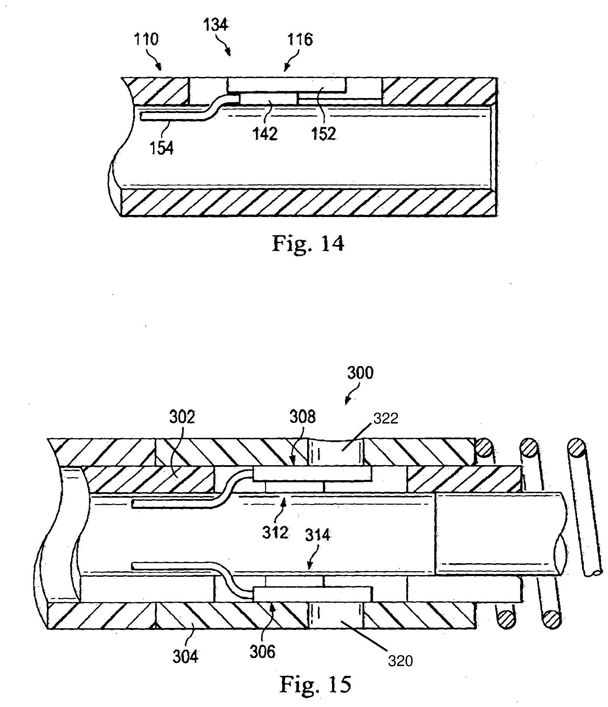

Fig. 14 shows the distal portion of the hypotube 110 with an alternative sensor assembly 116. This sensor assembly is similar in most respects to the sensor assembly discussed above, but includes conductors connected to the bottom or inner facing surface of the sensor block 152.

The proximal polymer sleeve 114 (Figs. 3 and 4) is disposed about the hypotube 110 and extends proximally from the sensor mount 134 toward the proximal electrical interface 122. In the exemplary embodiment shown, the polymer sleeve 114 is formed of a biocompatible polymeric material, such as Pebax®, for example, in order to reduce friction incurred as the guidewire is introduced through vessels in the body. Other materials may be used. Depending on the embodiment, the polymer sleeve 114 may have a thickness of about 0.0254 to 0.0508 mm (0.001 to 0.002 inch), although other thicknesses are contemplated. The sleeve 114 may include a hydrophilic coating that also lubricates and enables low friction passage through the vessels.

The distal tip 118 includes a coil 170, a flex element 172, and a distal cap 174. The coil 170 may be best seen in Figs. 3 and 4 and extends from the distal end region 132 of the hypotube 110 in the distal direction to the distal cap 174. As such, the coil 170 includes a distal portion 176 and a proximal portion 178. The coil 170 may be a coil spring formed of a suitable material such as stainless steel or Nitinol, for example. In one embodiment, the coil 170 has an inner diameter matching that of the outer diameter of the hypotube 110. The proximal portion 178 is connected or attached, such as by threading, onto the distal end region 132 of the hypotube 110. The distal portion 176 of the coil 170 is secured about the distal cap 174. In some embodiments, the coil 170 is formed of a highly radiopaque material such as palladium or a tungsten platinum alloy. In some examples, it has a length within a range of about 2cm to 3cm, although other ranges are contemplated.

The flex element 172 extends within an inner diameter of the coil 170 from the distal end region 132 of the hypotube 110. In the exemplary embodiment shown, the flex element 172 cooperates with the sensor mount 134 to be secured in place. The flex element 172 may be formed of any material suitable for bending while providing structural stability to the coil 170, including for example, a stainless steel wire, a Nitinol wire, or other biocompatible material.

The flex element 172 is formed of a body extending between and connecting a proximal end and a distal end. The flex element 172 flexes in order to traverse tortuous vessels in the patient's body. The body tapers from the proximal end to the distal end. Since the cross-section of the tapering body decreases in the distal direction, the distal end has a greater flexibility than the proximal end. As such, the flex element 172 may provide some stability and transition from more flexible in the distal direction to more stiff in the proximal direction. In the embodiment shown, the tapering body is cylindrically shaped, thereby forming a conical taper. Other embodiments have other profiles. For example, some embodiments have a square cross-section, a rectangular cross-section, an oval cross-section, or other shape.

The distal cap 174 is disposed over the coil 170 and the flex element 172 as shown in Fig. 3. In the example shown, the distal cap 174 has a leading rounded end that can smoothly slide against tissue as the guidewire 100 is fed through the vasculature of a patient. In this example, the distal cap 174 is a solder joint with a rounded end. In other embodiments, the distal cap 174 is a separate component secured to the coil 170 via an adhesive. However, in other embodiments, the distal cap 174 is secured to the coil 170 via welding or other attachment method.

The sensor housing 112 is disposed at the end of the polymer sleeve 114 and is configured to cover and protect the sensor assembly 116. As such, the sensor housing 112 covers the sensor mount 134. Since the stiffness of the hypotube 110 may be decreased by the sensor mount 134, the sensor housing 112 may be configured to restore the rigidity of the hypotube. In the embodiment shown, it does this by extending over and covering the cylindrical portions of the hypotube 110 at each end of the sensor mount 134, as can be seen in Fig. 4. The sensor housing 112 may be formed of a rigid material, such as a stainless steel, a nitinol alloy, or other biocompatible material that provides rigidity to the sensor mount region of the hypotube 110.

A window 196 in the sensor housing 112 provides fluid communication between the sensor assembly 116 in the sensor mount and the outer environment. In this embodiment, the window 196 is formed to lay directly above the sensor 150 and is sized and configured so that the detected physiological characteristic at the sensor equates to the environmental characteristic outside the hypotube. For example, when the sensor 150 is a pressure sensor, the window 196 is sized so that the pressure at the pressure sensor 150 is substantially the same as the pressure outside the sensor housing 112.

Some embodiments of the sensor housing 112 include a non-circular inner surface. Accordingly, the cross-section of the lumen may form an oval or other shape. In one embodiment, the oval shape accommodates sensor blocks that have a width greater than the outer profile of the hypotube with the sensor block is disposed on the sensor mount.

Fig. 15 shows an alternative embodiment of a guidewire 300. Because some of the elements of the guidewire 300 are the same as the guidewire 100 discussed above, for convenience, the descriptions of those elements will not be repeated. The guidewire 300 includes a hypotube 302, a sensor housing 304, and a plurality of sensor assemblies 306, 308. The hypotube 302 includes a plurality of integrated sensor mounts 312, 314. The embodiment in Fig. 15 includes two sensor mounts 312, 314 on opposing sides of the hypotube 302. Since the sensor mounts 312, 314 are formed in the walls of the hypotube 302 rather than in the lumen of the hypotube 302, the sensor assemblies 306, 308 may be disposed along the same axial region of the guidewire. That is, the sensor assemblies 306, 308 may be disposed about the same distance from the end of the hypotube 302. The sensor assemblies 306, 308 may include the same or different types of sensors. In one embodiment, the sensor assembly 306 is a pressure sensor and the sensor assembly 308 is a flow sensor. Any sensor type may be used for detecting a physiological condition of the patient. The sensor housing 304 in Fig. 15 includes a first window 320 associated with the sensor assembly 306 and includes a second window 322 associated with the sensor assembly 308.

Assembly of the guidewires may include obtaining the components or elements discussed above. In one embodiment, the integrated sensor mount 134 is formed in the hypotube 110 using a sinker EDM cutting process, although other methods may be used. The worker may introduce the flex element 172 into the sensor mount 134 so that the distal portion of the flex element 172 extends from the distal end of the hypotube 110. The flex element 172 may then be secured to the hypotube 110 by soldering or by using an alternative attachment method. Other attachment methods include, among others, adhesives and welding.

With the flex element 172 secured in the sensor mount 134, the sensor block 152 may be introduced to the sensor mount 134. The conductors 154 may be fed through the hypotube lumen to the sensor mount 134 to connect to the sensor block 152. The sensor block 152 carries the sensor 150 for detecting a physiological characteristic of a patient's vessel. As discussed above, in some embodiments, the sensor 150 is a pressure sensor.

The sensor block 152 may be lowered in the radial direction into the sensor mount until the inner facing surface of the sensor block 152 is mechanically stopped by the shelf 142. Lateral movement may be limited or prevented by the width of the sensor mount 134 and its relationship with the width of the sensor block 152. Accordingly, the lateral walls of the sensor mount 134 act as mechanical stops that limit or prevent lateral movement. As such, the sensor mount 134 includes mechanical stops that help guide sensor placement in at least two dimensions.

Because the sensor 150 lies directly on the shelf 142 forming a part of the hypotube sensor mount 134, variations in sensor height from guidewire to guidewire can be substantially reduced or eliminated. With the sensor height set by the sensor mount 134, and its lateral location set by the lateral walls of the sensor mount 134, the worker can further align the sensor block 152 by visually comparing the ends of the sensor block 154 relative to the ends of the sensor mount 134. Accordingly, the sensor mount 134 provides a mechanical stop or mechanical limit to aid a worker in consistently placing the sensor at the same height and at the same lateral position from guidewire to guidewire. In addition, the sensor mount 134 provides a guide in the form of edges of the mount that enables the worker to visually place the sensor block 152 in a desired location in the axial direction. Accordingly, the worker may be able to produce product with greater precision and consistency than in prior designs.

The sensor block 152 may be secured in place using an adhesive or other securing method, such as those discussed above. With the sensor block 152 now secured in place, the conductors 154 may be connected to the contacts 156 on the sensor block 152. In some embodiments, these are soldered to the contacts 156, however other attachment methods are contemplated to provide electrical communication. A sealant or adhesive may be used to isolate and protect the connections of the conductors 154 and the contacts 156.

The sensor housing 112 may then be introduced over the distal end of the hypotube 110 to cover the sensor mount 134 and to increase the rigidity of the hypotube 110 in the region of the sensor mount 134. The sensor housing 112 may be aligned so that its window overlies the sensor 150 and the distal and proximal ends lie upon the fully cylindrical portions at the distal and proximal sides of the sensor mount 134. The sensor housing 112 may be then secured to the hypotube using an adhesive or weld or other method.

With the sensor housing 112 and the flex element 172 in place on the hypotube 110, the coil 170 and the distal cap 174 may then be introduced to the hypotube 110. The distal cap 174 may be formed or soldered in place over the distal end of the coil 170 to form a rounded end. In embodiments where the distal cap 174 is a separate component, the distal cap may be secured using an adhesive, a weld, or other attachment method. In some aspects, the distal cap 174 is screwed or threaded onto the coil 170. With the distal cap on the coil 170, the coil may be introduced over the flex element 172 and secured to the hypotube 110. As discussed above, the coil may be secured by an adhesive, may be welded, soldered, or otherwise bonded to the hypotube 110. In some embodiments, the coil is threaded onto the hypotube.

Using the integrated sensor mounts disclosed herein may increase the repeatability and consistency of sensor placement during the manufacturing process. This may provide a more consistent product to the surgeons increasing surgeon satisfaction and simplifying the assembly process.

Persons skilled in the art will also recognize that the apparatus, systems, and methods described above can be modified in various ways. Accordingly, persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure.

## Claims

1. A guidewire system for treating a patient, comprising:
a sensor assembly (116,152) for detecting a physiological characteristic of a patient;
a hypotube (110) sized for insertion into vasculature of the patient and having an integrated sensor mount (134) therein for predictably locating the sensor during assembly, the hypotube (110) having a wall structure forming a lumen, the sensor mount (134) being formed within the wall structure of the hypotube (110) and having a first mechanical stop (142) configured to limit movement of the sensor assembly (116,152) in at least a first dimension and a second mechanical stop (w1) configured to limit movement of the sensor assembly (116,152) in at least a second dimension; and
a sensor housing (112) disposed about the sensor mount (134) and having a window (196) formed therein to provide fluid communication between the sensor assembly (116,152) and an environment outside the hypotube, and
wherein the first mechanical stop (142) is configured to maintain the sensor at a desired height, and wherein the second mechanical stop (w1) is configured to maintain the sensor at a desired lateral location, and
wherein the sensor mount (134) has a first region (138) having a first width (w1) and a second region (140) having a greater second width (w2), the sensor assembly (116,152) having a width corresponding to the first width (w1) of the first region (138) in a manner that substantially limits movement at the lateral location in a lateral direction, the sensor assembly (116,152) extending into the second region (140), the second width (w2) providing additional clearance for the sensor assembly (116,152) in a manner that prevents distortion of sensor readings when the hypotube (110) flexes as it traverses tortious vessels in the patient.

2. The guidewire system of claim 1, wherein the first mechanical stop (142) is a shelf (142) formed by the wall structure of the hypotube (110), the shelf being radially displaced (d1) from the outer surface of the hypotube (110); and
the sensor mount (134) comprises a through passage adjacent the shelf (142), the through passage being sized and configured to accommodate a portion of the sensor assembly (116,152) extending into a lumen of the hypotube when the sensor assembly is on the sensor mount.

3. The guidewire system of claim 1 or claim 2, wherein the second mechanical stop (w1) comprises lateral wall structure of the hypotube adjacent the sensor assembly (116,152).

4. The guidewire system of any preceding claim, wherein the integrated sensor mount (134) comprises a cutout having a first level (143) and a second level, the sensor assembly being disposed on the first level, the second level being lower than the first level.

5. The guidewire system of claim 2 or any of claims 3-4 when dependent on claim 2, wherein a portion of the sensor assembly extends off the shelf to a cantilevered position within the sensor mount.

6. The guidewire system of any preceding claim, wherein the hypotube is formed of Nitinol and the sensor housing is formed of stainless steel.

7. The guidewire system of any preceding claim, wherein the sensor mount is first sensor mount (312) and the sensor assembly is a first sensor assembly (308), the guidewire system comprising a second sensor mount (314) and a second sensor assembly (306).

8. A method of building a guidewire comprising:
providing a hypotube sized for introduction to a patient's vasculature when treating a medical condition, the hypotube having a sensor mount therein, the sensor mount formed within the wall structure of the hypotube and having a first mechanical stop configured to limit movement of the sensor assembly in at least a first dimension and a second mechanical stop configured to limit movement of the sensor assembly in at least a second dimension, wherein the sensor mount (134) has a first region (138) having a first width (w1) forming the second mechanical stop and a second region (140) having a greater second width (w2);
radially introducing a sensor assembly into the sensor mount in the hypotube, the sensor assembly (116,152) having a width corresponding to the first width (w1) of the first region (138) of the sensor mount, until the sensor assembly abuts a shelf recessed in a wall structure of the hypotube to locate the sensor assembly at a pre-established height by limiting movement of the sensor assembly beyond the shelf in the radial direction,
wherein radially introducing the sensor assembly also includes aligning the sensor assembly with the sensor mount so that lateral movement is substantially prevented in the first region (138) of the sensor mount and so that the sensor assembly extends into the second region (140), the second width (w2) providing additional clearance for the sensor assembly (116,152), in order to obtain consistency in sensor assembly placement from one guidewire to another.

9. The method of claim 8, further comprising extending a portion of the sensor assembly through a passage of the hypotube and into a lumen of the hypotube, wherein the portion of the sensor assembly is a plurality of conductors configured to communicate signals from a sensor to a proximal end of the guidewire.

10. The method of claim 8 or claim 9, further comprising introducing the sensor mount into a sensor housing having a window formed therein to provide fluid communication between the sensor and an environment outside the sensor housing.

## Patentansprüche

1. Ein Führungsdrahtsystem zur Behandlung eines Patienten, das Folgendes umfasst:
eine Sensoranordnung (116, 152) zum Erkennen einer physiologischen Eigenschaft des Patienten;
eine Hypotube (110) in einer zum Einführen in das Gefäßsystem des Patienten geeigneten Größe mit einer integrierten Sensorhalterung (134), um den Sensor während des Zusammenfügens vorhersagbar zu fixieren;
wobei die Hypotube (110) eine Wandstruktur aufweist, die ein Lumen bildet, und wobei sich die Sensorhalterung (134) in der Wandstruktur der Hypotube (110) befindet und einen ersten mechanischen Anschlag (142) aufweist, der die Bewegung der Sensoranordnung (116, 152) in mindestens einer ersten Dimension begrenzt. Zudem umfasst sie einen zweiten mechanischen Anschlag (w1), der die Bewegung der Sensoranordnung (116, 152) in mindestens einer zweiten Dimension begrenzt; und ein Sensorgehäuse (112), das um die Sensorhalterung (134) angeordnet ist und ein Fenster (196) aufweist, um eine Fluidverbindung zwischen der Sensoranordnung (116, 152) und der Umgebung außerhalb der Hypotube bereitzustellen, und
wobei der erste mechanische Anschlag (142) den Sensor in der gewünschten Höhe hält, und wobei der zweite mechanische Anschlag (w1) den Sensor in der gewünschten seitlichen Position hält, und
wobei die Sensorhalterung (134) einen ersten Bereich (138) mit einer ersten Breite (w1) und einen zweiten Bereich (140) mit einer größeren zweiten Breite (w2) aufweist, und wobei die Sensoranordnung (116, 152) eine Breite aufweist, die der ersten Breite (w1) des ersten Bereichs (138) in einer Weise entspricht, die an der seitlichen Position die Bewegung in seitlicher Richtung im Wesentlichen begrenzt, und wobei sich die Sensoranordnung (116, 152) in den zweiten Bereich (140) erstreckt, und wobei die zweite Breite (w2) für die Sensoranordnung (116, 152) einen zusätzlichen Freiraum bildet, der eine Verzerrung der Sensormesswerte verhindert, wenn sich die Hypotube (110) beim Durchqueren der gewundenen Gefäße des Patienten verbiegt.

2. Das Führungsdrahtsystem gemäß Anspruch 1, wobei es sich beim ersten mechanischen Anschlag (142) um einen von der Wandstruktur der Hypotube (110) gebildeten Sockel (142) handelt, und wobei der Sockel strahlenförmig von der Außenfläche der Hypotube (110) versetzt (d1) ist; und
wobei die Sensorhalterung (134) eine an den Sockel (142) angrenzende Durchführung umfasst, und wobei die Durchführung so bemessen und konfiguriert ist, dass sie den Teil der Sensoranordnung (116, 152) aufnehmen kann, der sich in ein Lumen der Hypotube erstreckt, wenn sich die Sensoranordnung an der Sensorhalterung befindet.

3. Das Führungsdrahtsystem gemäß Anspruch 1 oder 2, wobei der zweite mechanische Anschlag (w1) eine an die Sensoranordnung (116, 152) angrenzende Seitenwandstruktur der Hypotube umfasst.

4. Das Führungsdrahtsystem gemäß einem der vorherigen Ansprüche, wobei die integrierte Sensorhalterung (134) einen Ausschnitt mit einer ersten Ebene (143) und einer zweiten Ebene aufweist, wobei sich die Sensoranordnung auf der ersten Ebene befindet, und wobei sich die zweite Ebene unterhalb der ersten Ebene befindet.

5. Das Führungsdrahtsystem gemäß Anspruch 2 oder einem der Ansprüche 3 und 4, wenn diese abhängig von Anspruch 2 sind, wobei sich ein Teil der Sensoranordnung vom Sockel in eine ausgekragte Position innerhalb der Sensorhalterung erstreckt.

6. Das Führungsdrahtsystem gemäß einem der vorherigen Ansprüche, wobei die Hypotube aus Nitinol und das Sensorgehäuse aus rostfreiem Stahl besteht.

7. Das Führungsdrahtsystem gemäß einem der vorherigen Ansprüche, wobei es sich bei der Sensorhalterung um eine erste Sensorhalterung (312) und bei der Sensoranordnung um eine erste Sensoranordnung (308) handelt, und wobei das Führungsdrahtsystem eine zweite Sensorhalterung (314) und eine zweite Sensoranordnung (306) aufweist.

8. Eine Methode zum Bilden eines Führungsdrahts, die folgende Schritte umfasst:
Bereitstellen einer Hypotube in einer Größe, die sich beim Behandeln einer Erkrankung zum Einführen in das Gefäßsystem eines Patienten eignet, wobei die Hypotube eine integrierte Sensorhalterung aufweist, und wobei sich die Sensorhalterung in der Wandstruktur der Hypotube befindet und einen ersten mechanischen Anschlag aufweist, der die Bewegung der Sensoranordnung in mindestens einer ersten Dimension begrenzt. Zudem umfasst sie einen zweiten mechanischen Anschlag, der die Bewegung der Sensoranordnung in mindestens einer zweiten Dimension begrenzt, wobei die Sensorhalterung (134) einen ersten Bereich (138) mit einer ersten Breite (w1), der den zweiten mechanischen Anschlag bildet, sowie einen zweiten Bereich (140) mit einer größeren zweiten Breite (w2) aufweist;
strahlenförmiges Einführen einer Sensoranordnung in die Sensorhalterung der Hypotube, wobei die Sensoranordnung (116, 152) eine Breite aufweist, die der ersten Breite (w1) des ersten Bereichs (138) der Sensorhalterung entspricht, bis die Sensoranordnung an einen in die Wandstruktur der Hypotube eingelassenen Sockel stößt, um die Sensoranordnung in einer vorab festgelegten Höhe zu positionieren, indem die Bewegung der Sensoranordnung über den Sockel hinaus in radialer Richtung begrenzt wird,
wobei das strahlenförmige Einführen der Sensoranordnung zudem das Ausrichten der Sensoranordnung an der Sensorhalterung umfasst, sodass eine seitliche Bewegung im ersten Bereich (138) der Sensorhalterung im Wesentlichen verhindert wird und sich die Sensoranordnung in den zweiten Bereich (140) erstreckt, wobei die zweite Breite (w2) einen zusätzlichen Freiraum für die Sensoranordnung (116, 152) bildet, um eine konsistente Positionierung der Sensoranordnung von einem Führungsdraht zum anderen zu ermöglichen.

9. Die Methode gemäß Anspruch 8, die zudem Erweitern eines Teils der Sensoranordnung durch eine Durchführung der Hypotube sowie in ein Lumen der Hypotube umfasst, wobei es sich beim Teil der Sensoranordnung um mehrere Leiter handelt, die Signale vom Sensor zum proximalen Ende des Führungsdrahts übertragen.

10. Die Methode gemäß Anspruch 8 oder 9, die zudem das Einführen der Sensorhalterung in ein Sensorgehäuse mit einem Fenster umfasst, um eine Fluidverbindung zwischen dem Sensor und der Umgebung außerhalb des Sensorgehäuses bereitzustellen.

## Revendications

1. Système de fil-guide destiné au traitement d'un patient, comprenant :
un ensemble capteur (116, 152) pour détecter une caractéristique physiologique d'un patient ;
un hypotube (110) dimensionné pour être inséré dans le système vasculaire du patient et comportant un support de capteur (134) intégré à l'intérieur de celui-ci pour localiser de manière prévisible le capteur lors de l'assemblage, l'hypotube (110) comportant une structure de paroi formant une lumière, le support de capteur (134) étant formé à l'intérieur de la structure de paroi de l'hypotube (110) et comportant une première butée (142) mécanique conçue pour limiter le mouvement de l'ensemble capteur (116, 152) dans au moins une première dimension et une seconde butée (w1) mécanique conçue pour limiter le mouvement de l'assemble capteur (116, 152) dans au moins une seconde dimension ; et
un boîtier de capteur (112) disposé autour du support de capteur (134) et comportant une fenêtre (196) formée à l'intérieur de celui-ci pour fournir une communication fluidique entre l'ensemble capteur (116, 152) et un environnement à l'extérieur de l'hypotube, et
dans lequel la première butée (142) mécanique est conçue pour maintenir le capteur à une hauteur souhaitée, et dans lequel la seconde butée (w1) mécanique est conçue pour maintenir le capteur à un emplacement latéral souhaité, et
dans lequel le support de capteur (134) comporte une première zone (138) présentant une première largeur (w1) et une deuxième zone (140) présentant une seconde largeur (w2) supérieure, l'ensemble capteur (116, 152) présentant une largeur correspondant à la première largeur (w1) de la première zone (138) d'une manière, laquelle limite sensiblement le mouvement à l'emplacement latéral dans une direction latérale, l'ensemble capteur (116, 152) s'étendant dans la seconde zone (140), la seconde largeur (w2) fournissant un dégagement supplémentaire pour l'ensemble capteur (116, 152) d'une manière, laquelle empêche la distorsion des lectures de capteur lorsque l'hypotube (110) fléchit lorsqu'il traverse des vaisseaux tortueux chez le patient.

2. Système de fil-guide selon la revendication 1, dans lequel la première butée (142) mécanique est une étagère (142) formée par la structure de paroi de l'hypotube (110), l'étagère étant déplacée (d1) radialement de la surface extérieure de l'hypotube (110) ; et
le support de capteur (134) comprend un passage traversant adjacent à l'étagère (142), le passage traversant étant dimensionné et conçu pour recevoir une partie de l'ensemble capteur (116, 152) s'étendant dans une lumière de l'hypotube lorsque l'ensemble capteur est sur le support de capteur.

3. Système de fil-guide selon la revendication 1 ou la revendication 2, dans lequel la seconde butée (w1) mécanique comprend une structure de paroi latérale de l'hypotube adjacente à l'ensemble capteur (116, 152).

4. Système de fil-guide selon l'une quelconque des revendications précédentes, dans lequel le support de capteur (134) intégré comprend une découpe comportant un premier niveau (143) et un second niveau, l'ensemble capteur étant disposé sur le premier niveau, le second niveau étant inférieur au premier niveau.

5. Système de fil-guide selon la revendication 2 ou l'une quelconque des revendications 3 à 4 lorsqu'elles dépendent de la revendication 2, dans lequel une partie de l'ensemble capteur s'étend depuis l'étagère jusqu'à une position en porte-à-faux à l'intérieur du support de capteur.

6. Système de fil-guide selon l'une quelconque des revendications précédentes, dans lequel l'hypotube est formé de nitinol et le boîtier de capteur est formé d'acier inoxydable.

7. Système de fil-guide selon l'une quelconque des revendications précédentes, dans lequel le support de capteur est un premier support de capteur (312) et l'ensemble capteur est un premier ensemble capteur (308), ledit système de fil-guide comprenant un second support de capteur (314) et un second ensemble capteur (306).

8. Procédé de construction d'un fil-guide comprenant :
la fourniture d'un hypotube dimensionné pour être introduit dans le système vasculaire d'un patient lors du traitement d'une affection médicale, l'hypotube comportant un support de capteur à l'intérieur de celui-ci, le support de capteur étant formé dans la structure de paroi de l'hypotube et comportant une première butée mécanique conçue pour limiter le mouvement de l'ensemble capteur dans au moins une première dimension et une seconde butée mécanique conçue pour limiter le mouvement de l'ensemble capteur dans au moins une seconde dimension, dans lequel le support de capteur (134) comporte une première zone (138) présentant une première largeur (w1) formant la seconde butée mécanique et une seconde zone (140) présentant une seconde largeur (w2) supérieure ;
l'introduction radiale d'un ensemble capteur dans le support de capteur dans l'hypotube, l'ensemble capteur (116, 152) présentant une largeur correspondant à la première largeur (w1) de la première zone (138) du support de capteur, jusqu'à ce que l'ensemble capteur bute contre une étagère en retrait dans une structure de paroi de l'hypotube pour placer l'ensemble capteur à une hauteur prédéfinie par limitation du mouvement de l'ensemble capteur au-delà de l'étagère dans la direction radiale,
dans lequel l'introduction radiale de l'ensemble capteur comprend également l'alignement de l'ensemble capteur avec le support de capteur de telle sorte qu'un mouvement latéral est sensiblement empêché dans la première zone (138) du support de capteur et de telle sorte que l'ensemble capteur s'étend dans la seconde zone (140), la seconde largeur (w2) fournissant un dégagement supplémentaire pour l'ensemble capteur (116, 152) pour obtenir une cohérence dans le placement de l'ensemble capteur d'un fil-guide à un autre.

9. Procédé selon la revendication 8, comprenant en outre l'extension d'une partie de l'ensemble capteur à travers un passage de l'hypotube et dans une lumière de l'hypotube, dans lequel la partie de l'ensemble capteur est une pluralité de conducteurs conçus pour communiquer des signaux d'un capteur à une extrémité proximale du fil-guide.

10. Procédé selon la revendication 8 ou la revendication 9,
comprenant en outre l'introduction du support de capteur dans un boîtier de capteur comportant une fenêtre formée à l'intérieur de celui-ci pour fournir une communication fluidique entre le capteur et un environnement à l'extérieur du boîtier de capteur.
